Europäisches Patentamt

European Patent Office

Office européen des brevets

(11) Veröffentlichungsnummer: **0 158 037**
**A1**

(12) # EUROPÄISCHE PATENTANMELDUNG

(21) Anmeldenummer: **85101487.8**

(51) Int. Cl.⁴: **A 61 M 1/00**

(22) Anmeldetag: **12.02.85**

(30) Priorität: **15.02.84 DE 3405403**

(43) Veröffentlichungstag der Anmeldung:
**16.10.85 Patentblatt 85/42**

(84) Benannte Vertragsstaaten:
**BE DE FR GB IT NL**

(71) Anmelder: **Stöckert Instrumente GmbH**
**Osterwaldstrasse 10**
**D-8000 München 40(DE)**

(72) Erfinder: **Stöckert, Friedemann**
**St. Emmeran 53**
**D-8000 München 81(DE)**

(72) Erfinder: **Heinze, Werner**
**Höhenstrasse 6**
**D-8911 Windach(DE)**

(72) Erfinder: **Kreuzer, Rudolf**
**Kreillerstrasse 7/6**
**D-8000 München 80(DE)**

(74) Vertreter: **Wuesthoff, Franz, Dr.-Ing. et al,**
**Patentanwälte Wuesthoff -v. Pechmann-Behrens-Goetz**
**Schweigerstrasse 2**
**D-8000 München 90(DE)**

(54) **Blutabsaugevorrichtung.**

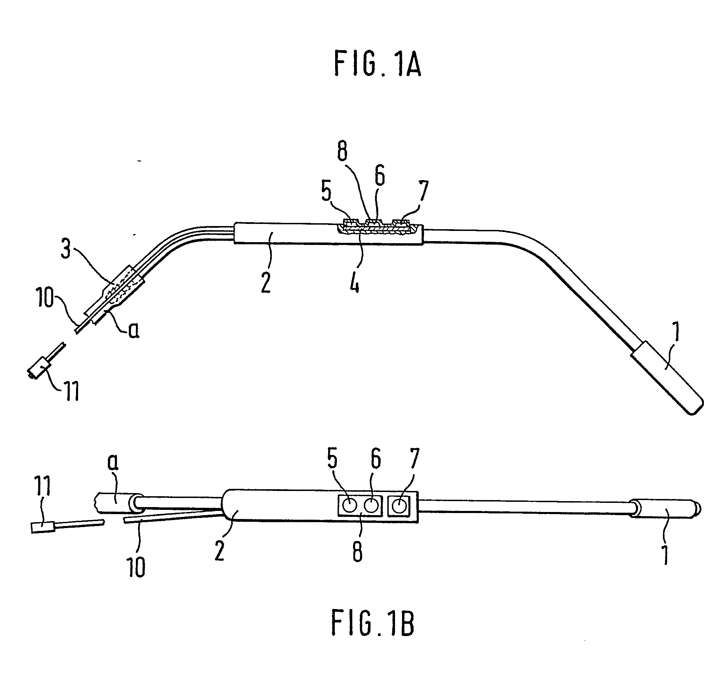

(57) Bei einer Blutabsaugevorrichtung zum Absaugen von Blut während eines chirurgischen Eingriffes, insbesondere im Herz- und Thorax-Bereich, mit einem Handgriff (2), einer daran befestigten Saugspitze (1) und einer die Saugspitze beaufschlagenden Vakuumleitung (a) zu einer Vakuumpumpe ist zur Änderung der Blut-Saugleistung an dem Handgriff (2) ein manuell betätigbares Betätigungsglied (7) vorgesehen, mit dem die Saugleistung der Vakuumpumpe veränderbar ist (Fig. 1a).

FIG. 1A

EP 0 158 037 A1

PATENTANWÄLTE

WUESTHOFF - v. PECHMANN - BEHRENS - GOETZ

EUROPEAN PATENT ATTORNEYS

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING. DIPL.-WIRTSCH.-ING. RUPERT GOETZ

D-8000 MÜNCHEN 90
SCHWEIGERSTRASSE 2
TELEFON: (089) 66 20 51
TELEGRAMM: PROTECTPATENT
TELEX: 5 24 070
TELEFAX: VIA (089) 2 71 60 63 (III)

Stöckert Instrumente GmbH
EP-58 565

## Blutabsaugevorrichtung

Die Erfindung betrifft eine Blutabsaugevorrichtung zum Absaugen von Blut während eines chirurgischen Eingriffs, insbesondere im Herz- und Thorax-Bereich, mit einem Handgriff, einer daran befestigten Saugspitze und einer die Saugspitze beaufschlagenden Vakuumleitung zu einer Vakuum-Pumpe, wobei die Blut-Saugleistung mittels zumindest eines am Handgriff angeordneten, manuellen Betätigungsgliedes einstellbar ist.

Nachdem bei einer Herzoperation mit extracorporalem Kreislauf zur Füllung des Schlauchvolumens, Oxygenators usw. das Patientenblut ohnehin durch Zugabe von Lösung verdünnt sowie durch Addition von Fremdblut ergänzt wird, besteht die Forderung, daß das im Operationsfeld abgesaugte Blut nicht vernichtet, sondern dem Kreislauf wieder zugeführt wird, weil einmal die Verdünnung nicht beliebig weit fortgesetzt werden kann und zum anderen die Zugabe von Fremdblut aus bekannten Gründen so gering wie möglich gehalten werden soll. Diese Tatsache erfordert es, daß der Qualität des durch Saugung zurückgewonnenen Blutes höchste Aufmerksamkeit zu widmen ist, das heißt die Traumatisierung dieses Blutes ist so gering wie möglich zu halten.

Läßt man die Saugerpumpe ständig laufen, um dem Operateur bei Bedarf sofort den Abtransport des Blutes zu ermöglichen, dann wird während des größten Teils der Operation Luft gesaugt, da nicht ständig extravasales Blut anfällt. Weil sich dabei wäh-

0158037

rend der Intervalle der Luftansaugung an der Saugerspitze kein Unterdruck aufbauen kann, verbleibt eine beträchtliche Menge Blut im Saugerschlauch, auf das die Pumpe ununterbrochen eingreift und dadurch in stets zunehmendem Maße vornehmlich Erythrozyten zerstört, umsomehr, je höher die Saugleistung eingestellt ist. Hält man die Sauganlage dagegen während der Zeit, in der kein Blut abgesaugt werden muß, ausgeschaltet, so ist dazu eine ständige mündliche Absprache zwischen Operateur und dem Bediener der Herz-Lungen-Maschine notwendig. Da letzterer keinen Einblick ins Operationsfeld hat und somit auf einen bevorstehenden Einschaltbefehl nicht immer vorbereitet ist, ergeben sich oft Verzögerungen und Kommunikationsschwierigkeiten, die zu unnötiger zusätzlicher Unruhe während der Operation führen können. Eine weitere Schwierigkeit besteht darin, daß der Sauger sich an leicht verletzlichen Körpergeweben, wie z.B. Herzklappen festsaugen kann. Auch aus diesem Grunde sollte die Anlage bei Nichtbedarf gestoppt werden.

Eine Blutabsaugevorrichtung der eingangs genannten Art ist aus der US-PS 4 205 677 bekannt. Dort erfolgt eine Steuerung der Saugleistung am Handgriff in der Nähe der Saugspitze. Die Steuerung der Saugleistung bezieht sich dort aber auf die Änderung der Blut- Saugleistung, nicht aber auf die Leistung der Pumpe. Die Saugleistung bezüglich des Blutes wird dort dadurch gesteuert, daß ein Luftventil mehr oder weniger in die Vakuumleitung eingeschaltet wird. Bei einer derartigen Anordnung gelangt beim Ansaugen des Blutes auch Luft in die Leitung, so daß aus dem entstehenden Blut/Luft-Gemisch sodann die Luft wieder entfernt werden muß, bevor das Blut in den Körper rezirkuliert werden kann.

Aus der FS-PS 23 71 202 ist eine Blutabsaugevorrichtung bekannt, bei der die Pumpe automatisch mittels Elektroden gesteuert wird, welche elektrisch das Vorhandensein von Blut ermitteln. Diese Sensoren sprechen aber auch in Luft auf an ihnen haftende Restblutfilme an und bewirken überdies, daß auch beim Anliegen der Sensoren an Körpergewebe die Pumpe eingeschaltet wird, was gerade vermieden werden sollte.

Auch in der GB-PS 1 166 670 und der US-PS 3 834 388 sind Blutabsaugevorrichtungen beschrieben, bei denen u.U. Luft in das Blut gelangen kann, was vermieden werden sollte.

Die bekannten Lösungen erfordern entweder aufwendige und deshalb störende Manipulationen seitens des Operateurs oder haben hinsichtlich der Rezirkulation des Blutes die beschriebenen Nachteile.

Der Erfindung liegt die Aufgabe zugrunde, die eingangs genannte Blutabsaugevorrichtung derart zu verbessern, daß die Ansaugung des Blutes von einem das Operationsfeld beobachtenden Operateur selbst auf einfache Weise gesteuert werden kann, wobei eine Verringerung der Blutqualität im Absaugschlauch, insbesondere eine Bluttraumatisierung, vermieden werden soll.

Die erfindungsgemäße Lösung dieser Aufgabe ist mit ihren Ausgestaltungen in den Patentansprüchen gekennzeichnet.

Erfindungsgemäß wird also mittels eines am Handgriff der Saugspitze angeordneten Betätigungsgliedes manuell die Vakuum-Pumpe vom Operateur je nach Bedarf an- oder ausgestellt.

In einer bevorzugten Ausgestaltung der Erfindung ist vorgesehen, daß mittels des Betätigungsgliedes die Saugleistung der Pumpe auf verschiedene Werte wahlweise einstellbar ist. Auch können insgesamt zwei Betätigungsglieder vorgesehen sein, wobei das erste dem Ein- und Ausstellen der Pumpe dient, während mit dem zweiten Betätigungsglied die Saugleistung der Pumpe manuell veränderbar ist. Dabei läßt sich die Saugleistung der Pumpe in einer bevorzugten Ausgestaltung der Erfindung kontinuierlich einstellen.

Eine fehlerfreie Betätigung der Blutabsaugevorrichtung kann in einer weiteren Ausgestaltung der Erfindung dadurch gefördert werden, daß zwei manuell betätigbare Einstellglieder vorgesehen sind, wobei bei Betätigung des einen Einstellgliedes die Saugleistung der Pumpe erhöht und bei Betätigung des anderen Einstellgliedes die Saugleistung der Pumpe erniedrigt wird.

Die Blutabsaugevorrichtung ist bevorzugt mit einer austauschbaren Saugspitze versehen.

Damit der Chirurg sich bei einer Unterbrechung des Saugbetriebes nicht den zuletzt eingestellten Zustand der Blutabsaugevorrichtung merken muß, ist in einer Weiterbildung der Erfindung eine Speicherschaltung vorgesehen, mittels welcher die gerade eingestellte Saugleistung der Vakuum-Pumpe speicherbar ist. Eine besonders bequeme Handhabung wird dadurch erreicht, daß eine optische Anzeigeeinrichtung am Handgriff vorgesehen ist, welche den momentanen Pumpenzustand dem Chirurgen anzeigt.

Nachfolgend ist die Erfindung mit weiteren Einzelheiten anhand der Zeichnung näher beschrieben. Es zeigt:

Fig. 1a eine Seitenansicht einer erfindungsgemäßen Blutabsaugevorrichtung und

Fig. 1b eine Draufsicht auf die in Fig. 1a gezeigte Blutabsaugevorrichtung.

Die Blutabsaugevorrichtung weist eine in herkömmlicher Weise ausgestaltete Saugerspitze 1, einen Handgriff 2 und einen Schlauchansatz 3 auf. Auf den Schlauchansatz 3 wird ein

- 5 - 0158037

Schlauch a aufgeschoben, welcher zur Vakuumpumpe (nicht gezeigt) führt.

Im Handgriff 2 ist eine Leiterplatte 4 eingelassen, die zumindest ein Schaltelement aufweist. In dem hier dargestellten Ausführungsbeispiel der Erfindung sind am Handgriff 2 insgesamt drei Schaltelemente 5, 6 und 7 vorgesehen. Eine durchsichtige Schutzkappe 8 schützt die Leiterplatte 4 gegen Berührung mit Flüssigkeit oder Gewebe.

Mit den Schaltelementen 5, 6 und 7 sind die einzelnen Leiter eines mehradrigen, abgeschirmten Kabels 10 verbunden, welches am vom Handgriff abgekehrten Ende in einer herkömmlichen Steckvorrichtung 11 endet. Die Steckvorrichtung 11 wird an die Vakuum-Pumpe (nicht gezeigt) angeschlossen. Eines der drei Schaltelemente, nämlich das Schaltelement 7, aktiviert bei Betätigung die Vakuum-Pumpe und schaltet diese bei erneuter Betätigung wieder ab. Die beiden anderen Schaltelemente 5 und 6 dienen der Erhöhung bzw. der Reduzierung der Pump-Saugleistung, wobei jeweils die Erhöhung bzw. Reduzierung solange kontinuierlich fortschreitet, wie das zugehörige Schaltelement 5 bzw. 6 gedrückt bleibt.

Das zum Stecker 11 führende Kabel 10 kann sowohl im Schlauch a verlaufen (Fig. 1a), als auch getrennt vom Schlauch a (Fig. 1b). Die Saugspitze 1 ist austauschbar aufgesteckt.

Mittels einer am Handgriff 2 angeordneten optischen Anzeigeeinrichtung (nicht gezeigt), wie beispielsweise einer lichtemittierenden Diode, läßt sich der jeweilige Zustand der Pumpe anzeigen. Die Anzeige kann in einer einfachen Ausgestaltung darüber informieren, ob die Pumpe an- oder ausgeschaltet ist, wobei im erstgenannten Zustand die Anzeigeeinrichtung leuchtet, während sie im letztgenannten Zustand nicht leuchtet. Auch kann in einer aufwendigeren Ausgestaltung der Anzeigeeinrichtung die jeweilige Saugleistung der Pumpe digital oder auf andere Weise optisch angezeigt werden.

2064

PATENTANWÄLTE

# WUESTHOFF - v. PECHMANN - BEHRENS - GOETZ

EUROPEAN PATENT ATTORNEYS

DR.-ING. FRANZ WUESTHOFF
DR. PHIL. FRIDA WUESTHOFF (1927-1956)
DIPL.-ING. GERHARD PULS (1952-1971)
DIPL.-CHEM DR. E. FREIHERR VON PECHM
DR.-ING. DIETER BEHRENS
DIPL.-ING.; DIPL.-WIRTSCH.-ING RUPERT (

D-8000 MÜNCHEN 90
SCHWEIGERSTRASSE 2

TELEFON: (089) 66 20 51
TELEGRAMM: PROTECTPATENT
TELEX: 524070
TELEFAX: VIA (089) 271 60 63 (III)

Stöckert Instrumente GmbH
EP-58 565

# P a t e n t a n s p r ü c h e :

1.    Blutabsaugevorrichtung zum Absaugen von Blut während eines chirurgischen Eingriffs, insbesondere im Herz- und Thoraxbereich, mit einem Handgriff (2), einer daran befestigten Saugspitze (1) und einer die Saugspitze beaufschlagenden Vakuumleitung (a) zu einer Vakuumpumpe, wobei die Blut-Saugleistung mittels zumindest eines am Handgriff angeordneten, manuellen Betätigungsgliedes einstellbar ist, dadurch   g e k e n n z e i c h n e t  , daß mittels des Betätigungsgliedes (7) die Vakuumpumpe an- und ausstellbar ist.

2.    Blutabsaugevorrichtung nach Anspruch 1, dadurch   g e k e n n z e i c h n e t  , daß mittels des Betätigungsgliedes (7) die Saugleistung der Pumpe einstellbar ist.

3.    Blutabsaugevorrichtung nach Anspruch 1, dadurch   g e k e n n z e i c h n e t  , daß neben dem Betätigungsglied (7) zumindest ein weiteres, manuell betätigbares Einstellglied (5 bzw. 6) vorgesehen ist, mit welchem die Saugleistung der Pumpe einstellbar ist.

4.    Blutabsaugevorrichtung nach einem der Ansprüche 2 oder 3, dadurch   g e k e n n z e i c h n e t  , daß die Saugleistung der Pumpe kontinuierlich einstellbar ist.

5. Blutabsaugevorrichtung nach einem der vorhergehenden Ansprüche, dadurch gekennzeichnet , daß zwei manuell betätigbare Einstellglieder (5, 6) vorgesehen sind, wobei bei der Betätigung des einen Einstellgliedes die Saugleistung der Pumpe erhöht und bei Betätigung des anderen Einstellgliedes die Saugleistung der Pumpe erniedrigt wird.

6. Blutabsaugevorrichtung nach einem der vorhergehenden Ansprüche, dadurch gekennzeichnet , daß die Saugspitze (1) austauschbar ist.

7. Blutabsaugevorrichtung nach einem der vorhergehenden Ansprüche, dadurch gekennzeichnet , daß eine Speicherschaltung vorgesehen ist, mittels welcher die gerade eingestellte Saugleistung der Vakuumpumpe speicherbar ist.

8. Blutabsaugevorrichtung nach einem der vorhergehenden Ansprüche, dadurch gekennzeichnet , daß eine optische Anzeige für den Pump-Zustand am Handgriff (2) vorgesehen ist.

2064

1/1

## FIG.1A

## FIG.1B

## EINSCHLÄGIGE DOKUMENTE

EP 85101487.8

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | FR - A - 2 080 017 (LE BOUFFANT - LE GALL) <br> * Gesamt * | 1,6 | A 61 M 1/00 |
| Y | US - A - 3 590 820 (S.A. NEHRA) <br> * Gesamt * | 1,6 | |
| A | WO - A1 - 81/03 125 (BIOMEDICAL ENG. CORP.) <br> * Gesamt; insbesondere Fig. 4,5; Zusammenfassung; Patentanspruch 1 * | 1,2,4 | |
| A | US - A - 3 335 727 (V.T. SPOTO) <br> * Gesamt * | 1,2,4 | |
| D,A | US - A - 4 205 677 (W.R. ENGSTROM) <br> * Gesamt * | 1,6 | RECHERCHIERTE SACHGEBIETE (Int Cl 4) <br><br> A 61 M 1/00 <br> A 61 M 7/00 |
| D,A | FR - A1 - 2 371 202 (AGENCE NATIONALE) <br> * Gesamt * | 1 | |
| A | US - A - 2 733 713 (H.H. KABNICK) <br> * Gesamt; insbesondere Spalte 3, Zeile 65 - Spalte 4, Zeile 5 * | 1,6 | |

Der vorliegende Recherchenbericht wurde für alle Patentansprüche erstellt

| Recherchenort | Abschlußdatum der Recherche | Prüfer |
|---|---|---|
| WIEN | 15-06-1985 | LUDWIG |